# EUROPEAN PATENT APPLICATION

(11) **EP 4 721 747 A1**
(43) Date of publication of application: **08.04.2026**
(21) Application number: 24811207.0
(22) Date of filing: 24.05.2024
(51) Int. Cl.: A61K 31/7105, A61K 8/60, A61K 48/00, A61P 17/14, A61P 43/00, A61Q 7/00

(54) **HAIR PAPILLA CELL ACTIVATOR AND HAIR AGENT**

(30) Priority: 25.05.2023 JP 2023085976
(71) Applicant: Asfreya Inc., Tokyo 136-0082 (JP)
(72) Inventor: OCHIYA Takahiro, Tokyo 104-0053 (JP)
(74) Representative: J A Kemp LLP
(86) International application number: PCT/JP2024/019306
(87) International publication number: WO 2024/242203

(57) **Abstract**

An object of the present invention is to provide a new ingredient which activates a dermal papilla cell, and can be used, for example, for hair regrowth, hair growth, prevention of white hair, and the like.

A dermal papilla cell activator according to the present invention comprises at least one miR selected from the group consisting of miR-27a-3p, miR-140-5p, and miR-224-5p.

## Description

### [Technical Field]

The present invention relates to a dermal papilla cell activator and a hair tonic.

### [Background Art]

Thin hair, white hair, and the like are hair problems that may occur regardless of gender or age, and generally considered to be caused by, for example, stress, aging, diet, sleep, heredity, and other factors. In recent years, various active ingredients have been explored to address these problems, and compositions containing low molecular weight compounds and others have been reported (Patent Literature 1).

### [CITATION LIST]

### [Patent Literature]

[Patent Literature 1] Japanese Patent Application Laid-Open No. 2023-050280

### [Summary of Invention]

### [Technical Problem]

Accordingly, an object of the present invention is to provide a new ingredient which can activate a dermal papilla cell, and can be used, for example, for hair regrowth, hair growth, and prevention of white hair.

### [Solution to Problem]

To achieve the aforementioned object, a dermal papilla cell activator according to the present invention comprises at least one miRNA selected from the group consisting of miR-27a-3p, miR-140-5p, and miR-224-5p.

A hair tonic according to the present invention comprises the aforementioned dermal papilla cell activator according to the present invention.

### [Advantageous Effects of Invention]

The present inventors found that certain miRNAs can activate dermal papilla cells, leading to the completion of the present invention. The present invention can activate a dermal papilla cell, enabling, for example, hair growth, prevention of white hair, and the like.

### [Brief Description of Drawings]

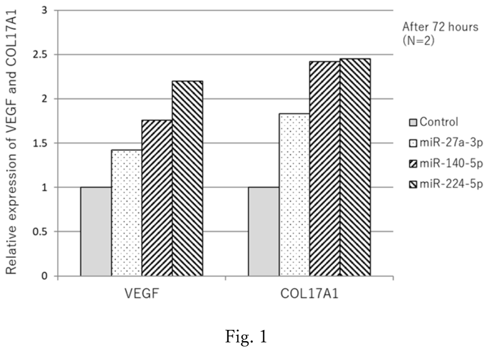

[Fig. 1] Fig. 1 shows graphs of Example 1 representing the expression amounts of the VEGF gene and the COL17A1 gene in dermal papilla cells in the presence of miRNAs.

### [Description of Embodiments]

[1] A dermal papilla cell activator comprising at least one miRNA selected from the group consisting of miR-27a-3p, miR-140-5p, and miR-224-5p.
[2] A hair tonic comprising the dermal papilla cell activator according to [1].
[3] The hair tonic according to [2], wherein the hair tonic is for hair growth and hair regrowth.
[4] The hair tonic according to [2] or [3], wherein the hair tonic is for preventing white hair.
[5] A method of activating a dermal papilla cell, the method comprising a step of administering to a scalp a dermal papilla cell activator comprising at least one miRNA selected from the group consisting of miR-27a-3p, miR-140-5p, and miR-224-5p.
[6] The method of activating a dermal papilla cell according to [5], wherein the administration to the scalp is performed transdermally or subcutaneously.
[7] At least one miRNA selected from the group consisting of miR-27a-3p, miR-140-5p, and miR-224-5p, wherein the at least one miRNA is used for activation of a dermal papilla cell.
[8] At least one miRNA selected from the group consisting of miR-27a-3p, miR-140-5p, and miR-224-5p, wherein the at least one miRNA is used for manufacture of a dermal papilla cell activator.

The terms used herein can be used in the senses commonly used in the art unless otherwise stated.

The term "improvement of hair" as used herein refers to hair-related phenomena in which the targets of the improvement are, for example, hair regrowth, hair growth, hair fostering, hair loss, hair graying, and the like. Hair regrowth means, for example, promoting regrowth of hair on the head, and hair growth means, for example, promoting growth of hair on the head, and hair fostering means, for example, inhibiting hair loss, protecting hair, and the like. The term "improvement" means, for example, maintenance of the present conditions, amelioration (making the conditions better, e.g., promoting), prevention of deterioration (inhibiting the conditions from getting worse), and the like. The meaning of the term "prevention of deterioration" includes, for example, inhibiting deterioration, delaying deterioration, and the like. In the present invention, the term "improvement of hair" also includes the meaning of improvement of scalp. In the present invention, the term "improvement" also includes the meaning of treatment. Treatment may mean, for example, inhibition of deterioration, palliation (amelioration), remission, or cure (complete recovery) of target symptoms, and may include "prevention" in a broad sense. The term "prevention" includes, for example, the meaning of inhibiting or delaying development of target symptoms and the like. When the term "treatment" is used herein in a narrow sense, the term "treatment" in the present specification may be interpreted as, for example, prevention.

Although the present invention will be described below with reference to specific examples, the present invention shall not be limited to those examples. Further, those exemplified in each embodiment of the present invention can each be referred to each other.

### (1) Dermal papilla cell activator

As described above, a dermal papilla cell activator according to the present invention comprises at least one miRNA selected from the group consisting of miR-27a-3p, miR-140-5p, and miR-224-5p. In the present invention, miR-27a-3p, miR-140-5p, and miR-224-5p are hereinafter referred to as the miRNAs of the present invention.

In the present invention, the activation of dermal papilla cells means, for example, a significant increase in the expression of at least one of the COL17A1 gene and the VEGF gene in the dermal papilla cells rather than the dermal papilla cells in the absence of the miRNAs of the invention. The COL17A1 gene and the VEGF gene are each known as a gene involved in hair growth, hair regrowth, and prevention of white hair.

COL17A1 (Gene Symbol) has a Gene Name of collagen type XVII alpha 1. VEGF has a Gene Symbol of VEGFA and a Gene Name of vascular endothelial growth factor A.

miR-27a-3p, miR-140-5p, and miR-224-5p are microRNAs (miRNAs). These three types of miRNAs are registered, for example, in the data base (MiRBase: https://www.mirbase.org) under the following Accessions Nos.

| | |
|---|---|
| miR-27a-3p | Accession No. MIMAT0000084 |
| miR-140-5p | Accession No. MIMAT0000431 |
| miR-224-5p | Accession No. MIMAT0000281 |

The aforementioned miRNAs, sequences of which are known, can be synthesized, for example, by methods of organic chemistry or by methods of genetic engineering. The aforementioned miRNAs, for example, may or may not be modified chemically.

The aforementioned miRNAs of the present invention in the dermal papilla cell activator according to the present invention are, for example, preferably miRNAs in a state where they are not contained in vesicles of biological origin. They can also be referred to as free miRNAs. The dermal papilla cell activator according to the present invention may be, for example, of a form which includes the aforementioned miRNA(s) of the present invention and does not include vesicles of biological origin.

The dermal papilla cell activator according to the present invention comprises as an active ingredient at least one miR selected from the group consisting of miR-27a-3p, miR-140-5p, and miR-224-5p. The dermal papilla cell activator according to the present invention, for example, may comprise the aforementioned active ingredient(s) only or may comprise the aforementioned active ingredient(s) and another additive ingredient.

The dermal papilla cell activator according to the present invention, for example, may comprise any one of the aforementioned three types of miRNAs, or may comprise any combinations of two (a combination of miR-27a-3p and miR-140-5p, a combination of miR-27a-3p and miR-224-5p, or a combination of miR-140-5p and miR-224-5p), or may comprise all of the aforementioned three types of miRNAs. When two types of miRNAs or three types of miRs are used in combination, there is no particular limitation for the ratio of them.

The dermal papilla cell activator according to the present invention may comprise as an active ingredient(s) the aforementioned miR(s) of the present invention only, or may comprise the aforementioned miRNA(s) of the present invention and another dermal papilla cell activating ingredient. The aforementioned another dermal papilla cell activating ingredient may be any ingredient which can activate a dermal papilla cell, for example, a ingredient which is already known or an ingredient which will be known after the filing of the present application.

The dermal papilla cell activator according to the present invention can be used, for example, for improving the hair of a living body of a subject, and can be used as a hair tonic according to the present invention as a specific example.

The dermal papilla cell activator according to the present invention may be used, for example, for administration to a living body of a subject. The aforementioned living body may be, for example, human or non-human animals. The aforementioned non-human animals include, for example, mammals such as mouse, rat, dog, cat, monkey, rabbit, bovine, goat, and camel.

Administration to the aforementioned living body may be, for example, parenteral. Parenteral administrations include, for example, transdermal administration, subcutaneous administration, and the like. Transdermal administrations include, for example, application to a scalp, and subcutaneous administrations include sub-scalp injection (for example, injection to subcutaneous tissues) and the like.

There is no particular limitation for the dosage of the dermal papilla activator according to the present invention, but it is preferably administered in a pharmaceutically effective amount. The pharmaceutically effective amount can be determined based on, for example, the route of administration, the type of an improvement target of hair, the presence or absence of symptoms related to an improvement target, the severity of symptoms, age, gender, and the like. There is no particular limitation for the content of the aforementioned miRNA(s) of the present invention in the dermal papilla cell activator according to the present invention, but the content is preferably determined, for example, such that it is administered in a pharmaceutically effective amount.

As a specific example, when the dermal papilla cell activator according to the present invention is used for application to a scalp, the miRNA(s) of the present invention in a concentration of 0.1 to 100 nmol/L can be applied to the entire scalp, and the number of applications per day may be, for example, one. The application amount per day of the miRNA(s) of the present invention may be, for example, 0.1 to 100 nmol/L and the like over the entire scalp, and the number of applications per day may be, for example, one. The interval between applications may be, for example, once a week, once a month, or once every three months, considering the hair cycle. As described above, there is no particular limitation for the content of the miRNA(s) of the present invention in the dermal papilla cell activator according to the present invention, but the miRNA(s) can be blended, for example, so as to achieve the exemplified dosages. There is no particular limitation for the concentration of the miRNA(s) of the present invention in the dermal papilla cell activator according to the present invention, and, for example, it may be a concentration which can be used as is or may be diluted at the time of use.

The dermal papilla cell activator according to the present invention may comprise, for example, the aforementioned active ingredient(s) and another additive ingredient, as described above. The aforementioned additive ingredients include, for example, pharmaceutically acceptable ingredients and the like. Specific examples include, for example, excipients, carriers (base materials), and the like. The aforementioned excipients and the aforementioned carriers include, for example, water-based solvents such as water, physiological saline, and buffer; oil and fats such as soybean oil; Vaseline; alcohol such as glycerol; sugar such as maltose, dextrose, and dextrin; sugar alcohol such as xylitol; phospholipid; liposome; and the like. In addition, the aforementioned additive ingredients also include, for example, binders, disintegrating agents, surfactants, emulsifying agents, antioxidant agents, lubricants, wetting agents, thickening agents, stabilizing agents, UV shielding agents, antiseptic agents, preservatives, vitamins, minerals, coloring agents, and the like. The aforementioned additive ingredients may include, for example, DDS such as liposome formulations, specific examples of which include phospholipid nanoparticles.

There is no particular limitation for the dosage forms of the dermal papilla cell activator according to the present invention, but they can be suitably selected depending on the route of administration. Specific examples of the aforementioned dosage forms include, for example, topical agents (topical drugs), injectable agents, and the like. The aforementioned topical agents include, for example, transdermal agents, and the like. Transdermal agents are preferred as a form for application to scalps. The forms of the aforementioned topical agents include, for example, liquid, emulsion, gel, sol, ointment, and the like.

The dermal papilla cell activator according to the present invention may be, for example, a pharmaceutical composition. The aforementioned pharmaceutical composition may be, for example, a pharmaceutical product, a quasi-drug, or a health product.

### (2) Hair tonic

As described above, the hair tonic according to the present invention comprises the dermal papilla cell activator according to the present invention, and specifically comprises at least one miRNA selected from the group consisting of the miRNAs of the present invention, i.e., miR-27a-3p, miR-140-5 p, and miR-224-5p. The description for the aforementioned dermal papilla cell activator according to the present invention can be referred for the hair tonic according to the present invention.

The hair tonic according to the present invention can increase the expression of genes related to hair regrowth, hair growth, prevention of white hair, and the like as described above by virtue of inclusion of the aforementioned miRNA(s) of the present invention. Therefore, the hair tonic according to the present invention can be used, for example, to improve the conditions of hair. The hair tonic according to the present invention, which can improve the symptoms related to hair, can also be referred to as, for example, a hair improving agent or an agent for improving hair symptoms.

### (3) Method of activating dermal papilla cells and method of improving hair

A method of activating a dermal papilla cell according to the present invention includes contacting at least one miRNA selected from the group consisting of the aforementioned miRNAs of the present invention, i.e., miR-27a-3p, miR-140-5p, and miR-224-5p with a dermal papilla cell. In the present embodiment, the aforementioned miRNAs of the present invention can be considered, for example, as the aforementioned dermal papilla cell activator according to the present invention.

For example, the method of activating a dermal papilla cell according to the present invention may be used *in vitro* or *in vivo.*

For example, the method of activating a dermal papilla cell according to the present invention includes a step of allowing the hair papilla cell to co-exist with the aforementioned miRNA(s) of the present invention in a case of *in vitro.* The aforementioned step preferably includes allowing the aforementioned dermal papilla cell to co-exist with the aforementioned miRNA(s) of the present invention in a culture medium, and culturing the cell. There is no particular limitation for the types of the culture medium and the culture conditions, but the general conditions for the aforementioned dermal papilla cell can be used.

Alternatively, the method of activating a dermal papilla cell according to the present invention includes, for example, an administration step of administering the aforementioned miRNA(s) of the present invention to a living body in a case of *in vivo.* The aforementioned administration step may be, for example, oral or parenteral, preferably parenteral. The aforementioned parenteral administration includes, for example, transdermal administration and subcutaneous administration. Examples of transdermal administration include, for example, application to a scalp, and examples of subcutaneous administration include, for example, sub-scalp injection (e.g., injection into subcutaneous tissues). For example, the aforementioned parenteral administration is preferably application to a scalp. There is no particular limitation for the conditions for administering the aforementioned miRNA(s) of the present invention, but those conditions exemplified for the dermal papilla cell activator according to the present invention can be used.

According to the present invention, a dermal papilla cell in a living body can be activated by administering the aforementioned miRNA(s) of the present invention to a living body. As a specific example, a dermal papilla cell of a living body can be activated by, for example, significantly increasing the expression of at least one of the COL17A1 gene and the VEGF gene in the dermal papilla cell.

The method of improving hair according to the present invention includes an administration step of administrating the aforementioned miRNA(s) of the present invention to a living body. The aforementioned miRNAs of the present invention can be considered, for example, as the aforementioned dermal papilla cell activator according to the present invention or the aforementioned hair tonic according to the present invention.

There is no particular limitation for the conditions for administering the aforementioned miRNA(s) of the present invention, but those conditions exemplified for the dermal papilla cell activator according to the present invention can be used. The method of improving hair according to the present invention can also be considered, for example, as a method of hair care, and the hair care includes scalp care.

According to the present invention, hair can be improved by administering the aforementioned miRNA(s) of the the present invention to a living body. The improvements of hair may be, for example, improvements in hair regrowth, hair growth, and white hair, and specific examples may be maintenance or amelioration of hair regrowth, maintenance or amelioration of hair growth, prevention of white hair, inhibition of white hair progression, and the like.

### (4) Use

The present invention relates to a use of at least one miR selected from the group consisting of miR-27a-3p, miR-140-5p, and miR-224-5p, wherein the at least one miR is used for activation of a dermal papilla cell. The present invention also relates to a use of at least one miR selected from the group consisting of miR-27a-3p, miR-140-5p, and miR-224-5p, for manufacturing a dermal papilla cell activator.

The present invention relates to a use of at least one miR selected from the group consisting of miR-27a-3p, miR-140-5p, and miR-224-5p, wherein the at least one miR is used for improvement of hair. The present invention also relates to a use of at least one miR selected from the group consisting of miR-27a-3p, miR-140-5p, and miR-224-5p, for manufacturing a hair tonic.

Although the present invention will be described below in detail with reference to Examples and the like, the present invention shall not be limited to these.

### [Examples]

### [Example 1]

The miRNAs of the present invention: miR-27a-3p, miR-140-5p, and miR-224-5p were investigated for increased expression of the COL17A1 gene and the VEGF gene.

### (Reagents and conditions)

Human papilla cells (Human Follicle Dermal Papilla Cells; HFDPC): Product name: 602-05A, Sigma
Culture medium: Hair Follicle Dermal Papilla Cell Growth Medium, Product name: 611-500, a dedicated medium, Sigma
A flask coated with collagen: Product name; Collagen Coated T-75-Flask (125-75), Corning.
Culture conditions: 37°C, 5% CO₂, 95% Air

First, HFDPC was pre-cultured for 72 hours in 6 mL of the aforementioned culture medium in a T-25 flask, and 6 mL of the pre-cultured culture was added to the aforementioned flask having 6 mL of a flesh culture medium added. After 18 hours of culturing, one of the aforementioned miRNAs was then added so as to give a final concentration of 10 nmol/L in the culture medium. In order to transfect the cells with the aforementioned miRNA, a transfection reagent (Product name: JetPEI^{®}, PPU) was added according to the instructions when the aforementioned miRNA was added. After the addition of the aforementioned miRNA, HFDPC was cultured for additional 72 hours and then collected (N=2).

RNAs were then extracted from the collected HEDPC, and the expression amounts of the COL17A1 gene (Assay ID: Hs00990076_g1) and the VEGF gene (Assay ID: Hs00900054_m1) were determined by TaqMan^{®} Gene Expression Assays. It is noted that the expression amount of each gene was also determined for the control HEDPC (N=2) cultured in a similar way except that the miRs were absent. The expression amount of each gene in the cells cultured in the presence of each miR was determined as a value relative to the expression amount of each gene in the control set as 1. These results are shown in graphs of Fig. 1.

Fig. 1 shows graphs representing the relative values of the expression amounts of the COL17A1 gene and the VEGF gene. The upper graph shows the relative expression amount of the COL17A1 gene, and the lower graph shows the relative expression amount of the VEGF gene. The vertical axis in each graph represents relative expression amounts. As shown in Fig. 1, an increase in expression was observed for the both genes when culturing HEDPC in the presence of each miR. Among these, miR-140-5p and miR-224-5p showed relatively high expression amounts, especially miR-224-5p showed a marked increase in expression amounts. These genes are known to be involved in hair regrowth, hair growth, and prevention of white hair. Therefore, these results demonstrate that each miR of the present invention can promote hair regrowth, hair growth, and prevention of white hair by promoting the expression of these genes.

Although the present invention was described above with reference to the embodiments, the present invention shall not be limited to the above embodiments. Various alternations which can be envisioned by those skilled in the art may be made to the configurations and details of the present invention within the scope of the present invention.

This application claims the priority based on Japanese Patent Application No. 2023-85976 filed on May 25, 2023, the entire disclosure of which is incorporated herein.

### [Industrial Applicability]

The present invention can activate a dermal papilla cell, enabling, for example, hair regrowth, hair growth, prevention of white hair, and the like.

## Claims

1. A dermal papilla cell activator comprising at least one miRNA selected from the group consisting of miR-27a-3p, miR-140-5p, and miR-224-5p.

2. A hair tonic comprising the dermal papilla cell activator according to claim 1.

3. The hair tonic according to claim 2, wherein the hair tonic is for hair growth and hair regrowth.

4. The hair tonic according to claim 2 or 3, wherein the hair tonic is for preventing white hair.

5. A method of activating a dermal papilla cell, the method comprising a step of administering to a scalp a dermal papilla cell activator comprising at least one miRNA selected from the group consisting of miR-27a-3p, miR-140-5p, and miR-224-5p.

6. The method of activating a dermal papilla cell according to claim 5, wherein the administration to the scalp is performed transdermally or subcutaneously.

7. At least one miRNA selected from the group consisting of miR-27a-3p, miR-140-5p, and miR-224-5p, wherein the at least one miRNA is used for activation of a dermal papilla cell.

8. At least one miRNA selected from the group consisting of miR-27a-3p, miR-140-5p, and miR-224-5p, wherein the at least one miRNA is used for manufacture of a dermal papilla cell activator.
